# EUROPEAN PATENT SPECIFICATION

(11) **EP 1 421 907 B1**
(45) Date of publication and mention of the grant of the patent: **06.10.2010**
(21) Application number: 02755872.5
(22) Date of filing: 08.08.2002
(51) Int. Cl.: A61B 10/00

(54) **Bone marrow harvesting set**
Set zur Entnahme von Knochenmark
Ensemble pour échantillonnage de moelle osseuse

(30) Priority: 09.08.2001 JP 2001241586; 13.06.2002 JP 2002172476
(43) Date of publication of application: 26.05.2004
(73) Proprietor: Jimro Co., Ltd., Takasaki-shi, Gunma 370-0021 (JP)
(72) Inventor: IKEHARA, Susumu, Osaka-shi, Osaka 534-0016 (JP); NAKAMURA, Shuji, Kawasaki-shi, Kanagawa 214-0038 (JP); AOKI, Masato SENKO MEDICAL INSTRUMENT MFG. CO.,LTD, Bunkyo-ku, Tokyo 113-0033 (JP); NUMAZAWA, Masaaki, Bunkyo-ku, Tokyo 113-0033 (JP); ADACHI, Masakazu, Takasaki-shi, Gunma 370-0864 (JP)
(74) Representative: HOFFMANN EITLE
(86) International application number: PCT/JP2002/008101
(87) International publication number: WO 2003/015637

(56) References cited:
- JP-A- 4 338 470
- JP-A- 11 504 234
- JP-A- 2001 025 469
- JP-B2- 62 007 849
- US-A- 4 915 255
- US-A- 5 913 859
- US-A- 6 071 284
- US-A1- 2001 009 978
- US-B1- 6 248 110

## Description

### BACKGROUND OF THE INVENTION

### Field of the invention

This invention relates to a bone marrow harvesting set for harvesting bone marrow cells aseptically using a bone marrow harvesting needle which is kept indwelling a bone to be used for, for example, harvesting bone marrow, in particular, to a bone marrow harvesting needle used for drilling a hard bone, such as long bone.

### Description of the related art

Bone marrow has been harvested using a bone marrow puncture needle, such as Thomas needle, to puncture ilium. The bone marrow puncture needle is constructed in such a manner as to insert and fit an inner needle into a tubular mantle while allowing the tip of the inner needle to project from the mantle. The bone marrow puncture needle, which is equipped with a handle, percutaneously punctures ilium, and once the tip of the needle reaches bone marrow, the inner needle alone is drawn out and the mantle is kept indwelling the bone marrow to be subsequently used.

As a method for harvesting bone marrow using this bone marrow puncture needle, typically, an aspiration method is employed. The aspiration method is such that the mantle of the bone marrow puncture needle is connected to a syringe to collect bone marrow by virtue of the aspiration force. In this method, however, since the quantity of bone marrow harvested from one site of a bone is as small as several ml, collecting a sufficient quantity of bone marrow requires a number of sites to be punctured; consequently, the method carries a high risk of allowing peripheral blood T cells to mix into the harvested bone marrow, and hence of causing GVHD, which necessitates immunosuppression.

Under these circumstances, a bone marrow perfusion method, as a method for overcoming the above problems, has been developed. The bone marrow perfusion method is such that two bone marrow harvesting needles are kept indwelling respective ends of a long bone, such as humerus, with one of the needles connected to an injection syringe and the other connected to a centrifugal tube for harvesting bone marrow via a collection tube, so as to perfuse bone marrow with a medium such as sterilized and heparinized phosphate-buffered physical saline, and thereby a required quantity of bone marrow is harvested into a collection container at a time by injecting the medium slowly into the bone marrow in such a manner as to wash away the bone marrow.

The conventionally employed method in which a bone marrow puncture needle, equipped with a handle, is pressed into a bone by turning the handle by hand is not suitably applied to the bone marrow perfusion method, because the method using a handle does not easily allow the tip of the needle to reach the cavity accommodating bone marrow.

Thus a method has been desired in which a long bone or the like is drilled with a bone marrow harvesting needle equipped with a drill which is capable of providing a more powerful rotational power than a handle.

Moreover, US-A-6,071,284 discloses a bone marrow harvesting set having a bone marrow harvesting needle comprising a connection chamber in which bone cuttings are collected. This collecting chamber is connected via a collection tube to a container for transferring the bone cuttings from the collecting chamber to the container. The collecting tube being attached at one end to the collecting chamber and, hence, the mantle of a bone marrow harvesting needle and at its other end to the container.

### SUMMARY OF THE INVENTION

This invention has been made in light of the above described problems. Accordingly, the object of the invention is to provide a bone marrow harvesting set capable of harvesting bone marrow with minimized exposure to a non-aseptic environment.

In order to accomplish the object, a bone marrow harvesting set is provided which includes the features of claim 1.

Desirably, the cap is provided with an air vent to equalize the internal and external pressures of the centrifuge container and the air vent is mounted with a filter.

Preferably, the bone marrow harvesting needle used in combination with the set includes: a tubular mantle; and an inner needle, the inner needle being inserted into and detachably attached to the mantle with its tip allowed to project from the mantle, and the bone marrow harvesting needle is **characterized in that** the inner needle has a drilling edge formed at its tip and the mantle has a cutting edge at its tip.

Preferably a helical groove is formed on the periphery of the inner needle.

Preferably the face of the cutting edge is constructed in such a manner that it slopes upward from the inner periphery toward the outer periphery of the cutting edge so that fine cut pieces of a bone are gathered onto the inner needle along the cutting edge and discharged into the upper portion along the helical groove.

Preferably co-rotation preventive mechanism is provided for preventing the mantle from co-rotating with the inner needle.

Preferably the co-rotation preventive mechanism is constructed in such a manner that a rotational power produced by an independent rotation driving source is transmitted to the concentrically positioned inner needle and mantle and that the inner needle and the mantle are rotated at different angular velocities.

Preferably the co-rotation preventive mechanism comprises a speed reduction mechanism in which the mantle is rotated at a reduced speed by transmitting the rotation of the inner needle.

Preferably the inner needle is provided with a holder, which is to be gripped by chuck claws of a drill, and at the rear end of the holder a fitting portion is formed for fitting a handle.

The invention provides the following advantages.

The use of the bone marrow harvesting needle may realize the bone marrow collection by the bone marrow perfusion method quickly, because the inner needle and the mantle have a drilling edge and a cutting edge at their respective tips, allowing a hole to be drilled in a bone by just rotating them without undue force, unlike the use of the bone marrow puncture needle, and allowing the bone marrow harvesting needle to indwell even in a hard and thick bone, such as long bone, quickly and easily. Further, the use of the needle of the invention ensures the safety of donors and makes bone marrow collection less invasive, which may increase donors' cooperation.

The use of the bone marrow harvesting set of the invention may minimize the exposure of the harvested bone marrow to a non-aseptic environment and avoid chances of recipients' suffering from infectious diseases, because, when harvesting bone marrow, the cap is put on the centrifuge container, one of the connectors, fitted to one end of the collecting tube, is closely fitted into the inlet of the cap, and the other connector, fitted to the other end of the collecting tube, is closely fitted into the mantle of the bone marrow harvesting needle.

Thus, according to the invention, bone marrow cells may be harvested in a safe manner and in large quantities, and the bone marrow harvesting needle and the drill may also be used for intra-bone marrow - bone marrow transplantation (IBM-BMT) in which bone marrow cells are transplanted directly into the bone marrow cavity. Further, the invention may contribute not only to bone marrow transplantation, but also to organ transplantation (using bone marrow transplantation in combination), and to study of human mesenchymal stem cell and the regenerative medicine using the same.

If a helical groove is formed in the periphery of the inner needle, fine cutting pieces of a bone produced by drilling the bone may be discharged along the helical groove, resulting in improvement of bone drilling efficiency.

If co-rotation preventive mechanism for preventing the mantle from co-rotating with the inner needle is provided, discharging fine cutting pieces of a bone may be further ensured on the conveyance principle of a screw conveyer.

The other features of the invention will become more apparent from the following detailed description of preferred embodiments of the invention with reference to the drawings.

### BRIEF DESCRIPTION OF THE DRAWINGS

In the accompanying drawings,
FIG. 1(a) is a front view of the inner needle of a bone marrow harvesting needle of embodiment 1, FIG. 1(b) is a vertical sectional view of the mantle of the bone marrow harvesting needle, FIG. 1(c) is a cross sectional view showing the performance of inserting and fitting the inner needle into the mantle, and FIG. (d) is a cross sectional view of the bone marrow harvesting needle;
FIG. 2(a) is a partially cutaway front view of the mantle body of the bone marrow harvesting needle of FIG. 1, FIG. 2(b) is a front view, on an enlarged scale, of the tip portion of the mantle body, FIG. 2(c) is a bottom view, on an enlarged scale, of the tip portion of the mantle body, and FIG. 2(d) is a front view, on an enlarged scale, of the cutting edge portion of the mantle body;
FIG. 3(a) is a cross sectional view showing the state in which the bone marrow harvesting needle is attached to a manual handle, and FIG. 3(b) is a fragmentary cross sectional view, on an enlarged scale, showing the attached state;
FIG. 4 is a view showing the state in which the bone marrow harvesting needle is gripped and held by the chuck claws of the electric drill;
FIG. 5 is a view showing the structure of the electric drill;
FIGS. 6(a) and 6(b) illustrate a method for harvesting bone marrow using a guide needle;
FIG. 7 illustrates a method for harvesting bone marrow using a bone marrow harvesting set;
FIG. 8 is a front view of a bone marrow harvesting needle of embodiment 2;
FIG. 9(a) is a plan view of the lower housing of a mantle and FIG. 9(b) is a plan view of the upper housing of a mantle;
FIG. 10 is a perspective view illustrating the performance of assembling the mantle;
FIG. 11 is a vertical sectional view of the bone marrow harvesting needle of FIG. 8;
FIG. 12 is a cross sectional view taken on line A-A of FIG. 8;
FIG. 13 is a cross sectional view illustrating the performance of discharging cut pieces of a bone; and
FIGS. 14(a) and 14(b) are graphs showing the measured results of cutting force.

### DETAILED DESCRIPTION OF THE PREFERRED EMBODIMENTS

In the following the embodiments of the invention will be described with reference to the accompanying drawings.

### [Embodiment 1]

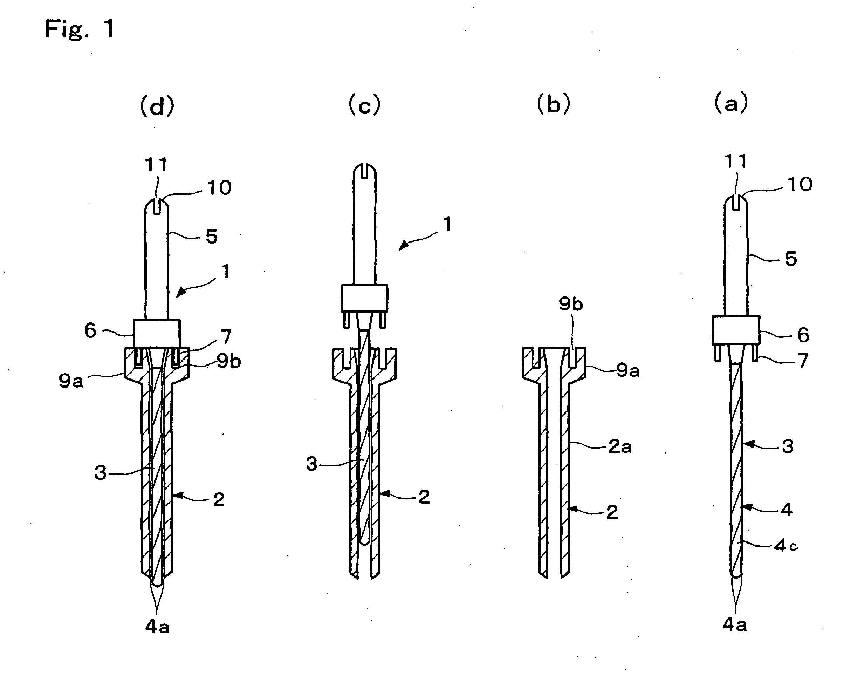

As shown in FIGS. 1(a) to 1(d), a bone marrow harvesting needle 1 consists of a mantle (outer needle) 2 and an inner needle 3 to be fitted into the mantle 2.

The inner needle 3 includes an inner needle body 4 and a holder 5, between the inner needle body 4 and the holder 5 an external flange 6 is formed, and on the external flange 6 a pair of pins 7 project to prevent free rotation of the mantle 2.

The inner needle body 4 forms a drill cutter having a cutting edge 4a at its tip. A helical groove 4c is formed throughout the full length of the inner needle body 4, and the tip of the inner needle body has a sharp cutting edge.

The mantle 2 includes a mantle body 2a and a fitting portion 9a, and the fitting portion 9a (e.g. lure lock type) is provided with a pair of pin holes 9b.

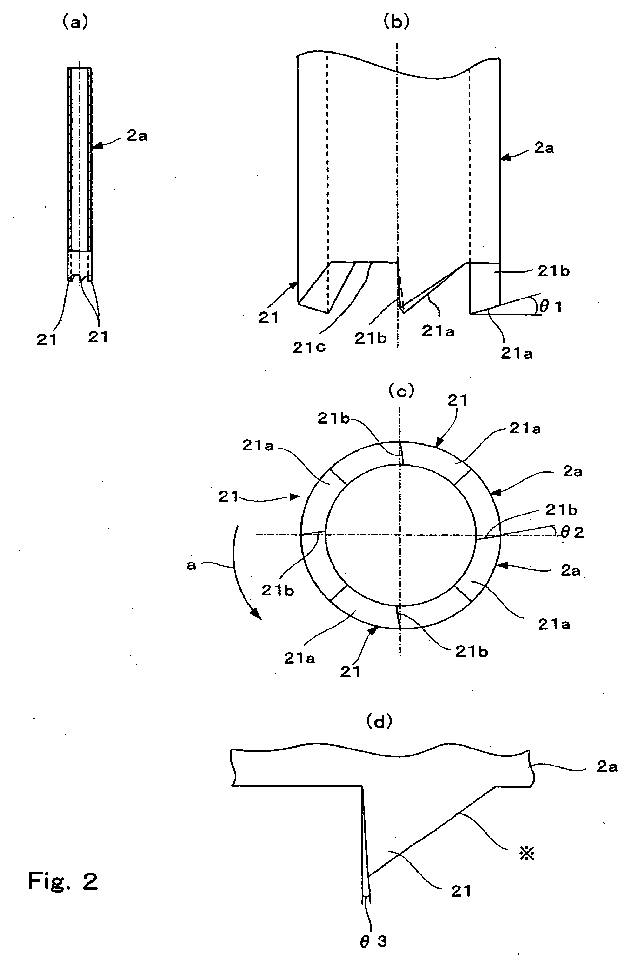

The mantle body 2a is cylindrically formed as shown in FIG. 2(a), and at the tip of the mantle body 2a a multiple of cutting edges 21 are formed at uniform intervals in a circumferential direction. On each cutting edge 21, flanks 21a and 21c are formed, and the flank 21a slopes radially inward in the downward direction at an angle θ1 (about 15 degrees), as shown in FIG. (b). The rake face 21b of the cutting edge slopes backward relative to the rotational direction in the radially inward direction at an angle θ2 (about 10 degrees), as shown in FIG. 2(c), so that cut pieces of a bone C2, described later, are emitted inside the mantle body 2a, and a front rake θ3 is set at a negative angle (about 5 degrees) such that a cutter tip slants backward as shown in FIG. 2(d). The arrow a in FIG. 2(c) indicates the rotational direction of the mantle body 2a.

When the inner needle 3 is fit into the mantle 2, the inner needle body 4 is inserted into the mantle body 2a in such a manner as to fit the pins 7 into the pin holes 9b and allow the tip portion of the inner needle body 4 to project from the tip of the mantle body 2a, as shown in FIGS. 1(c) and 1(d).

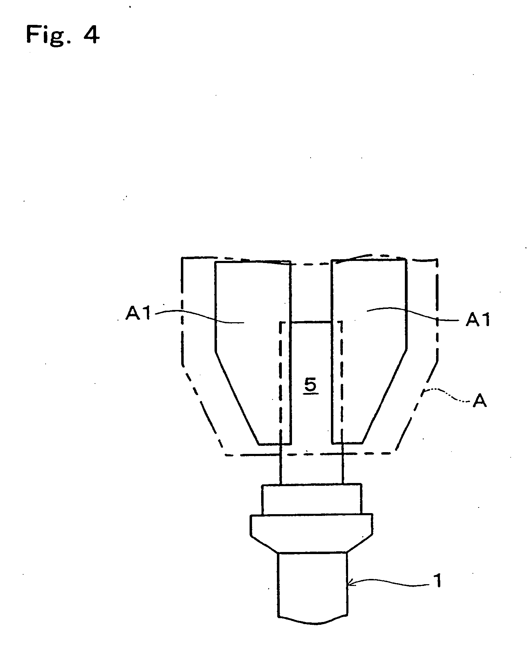

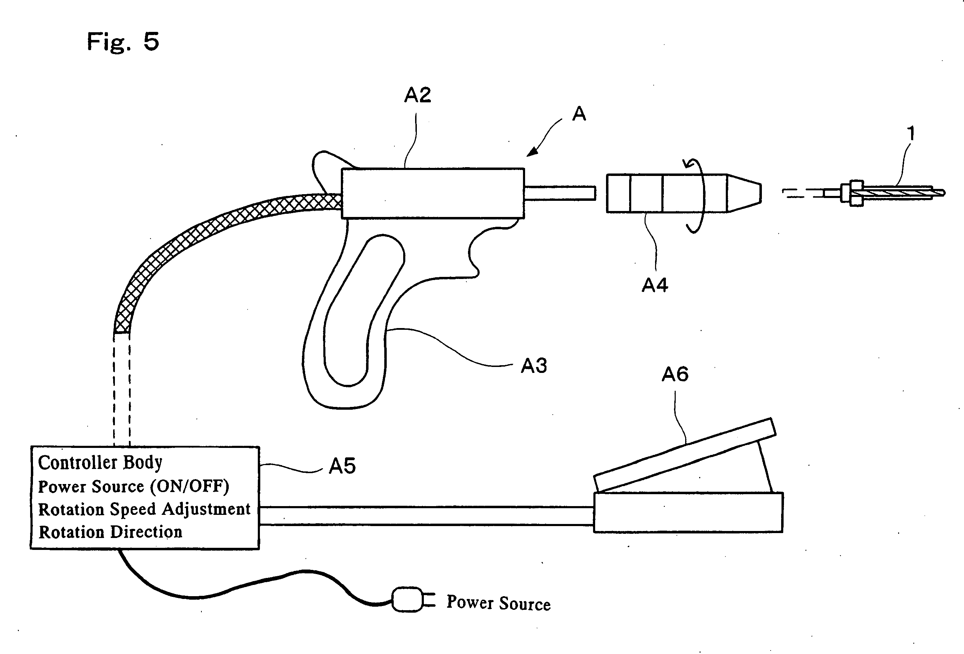

As shown in FIGS. 4 and 5, the bone marrow harvesting needle 1 can be rotated by an electric drill A with the holder 5 of its inner needle 3 being gripped by the chuck claws A1 of the electric drill A. The electric drill A includes: a motor portion A2; a grip handle portion A3; a chucking portion A4; a control portion A5 for controlling the rotational speed and direction; and a fit switch A6 for the motor of the motor portion A3. The drill A can be handled with one hand. Instead of the electric drill A, a nitrogen gas driving drill, for example, a model manufactured by Zimmer, which is used in the field of orthopedic surgery, can also be used.

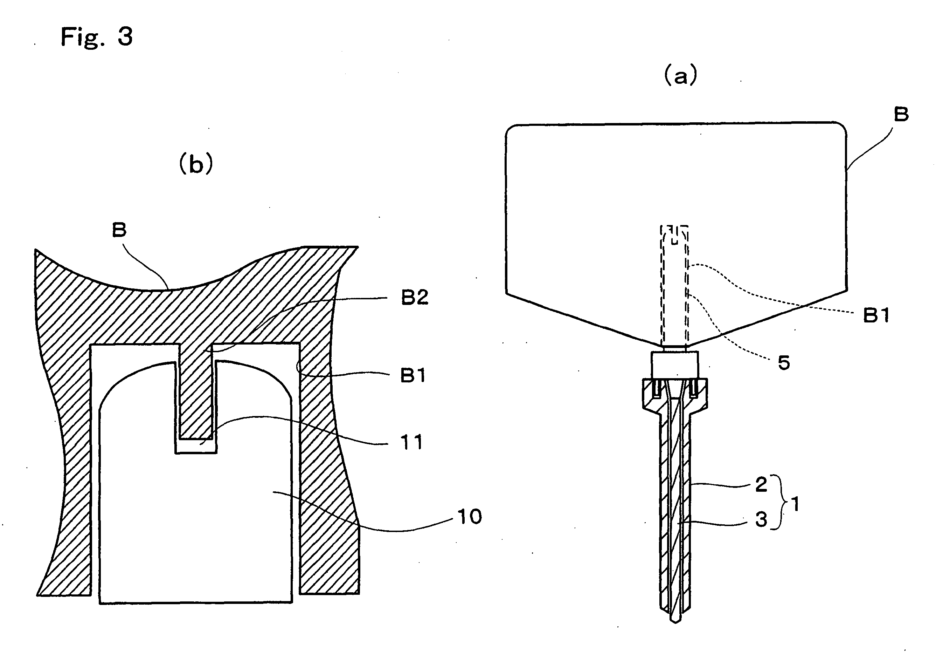

As shown in FIGS. 3(a) and 3(b), the bone marrow harvesting needle 1 is fitted into a handle B. A fitting portion 10 for fitting the needle into the handle B is formed at the back end of the holder 5 of the inner needle 3, as shown in FIG. 3(a). In the fitting portion 10, a narrow groove portion 11 is provided for preventing idling rotation of the handle B, whereas in the handle B a fitting hole B1 into which the fitting portion 10 is pressed is formed. Within the fitting hole B1, a idling rotation preventive piece B2 to be inserted into the groove portion 11 of the fitting portion 10 is provided. When using the handle B, the bone marrow harvesting needle 1 can be rotated by hand, which allows delicate manipulation of the needle 1 and makes more accurate and easier the operation at sites where the length of the needle 1 to be inserted into the cavity of bone marrow has to be regulated delicately.

An average size of the bone marrow harvesting needle 1 will be shown below as an example. In actuality, however, a set of several kinds of bone marrow harvesting needles different in length are prepared, since the distance from the skin surface to the periosteal surface differs from person to person. As to long needles, in order to maintain their mechanical strength, a balance of length and thickness shall be kept by making the outside diameters larger.

Average size of bone marrow harvesting needle 1 as an example:

| | |
|---|---|
| Outside diameter of mantle body 2a | 2.0 mm |
| Inside diameter | 1.5 mm |
| Length of mantle body 2a | 23.0 mm |
| Outside diameter of inner needle body 4 | 1.0 mm |
| Length of inner needle body 4 | 20.0 mm |
| Length of holder 5 of inner needle 3 | 20.0 mm |

As for recipients, who are the subjects of bone marrow transplantation, organ transplantation, regenerative medicine, etc. , their immunological functions are significantly lowered due to the pretreatment such as X-ray irradiation. Therefore, avoiding chances of their suffering from infectious diseases as much as possible is indispensable to treatments involving transplantation. In such a situation, the use of a bone marrow harvesting set, which will be described below, in the harvest of bone marrow may minimize the exposure of harvested bone marrow to a non-aseptic environment.

The bone marrow harvesting set includes: a rigid plastic centrifuge container E(with a volume of, for example, 60 ml); a cap H with an air vent H2, into which a filter H1 (made of, for example, sintered resin) is fitted, an collection inlet H3; and an collection tube F with connectors G fixed to both of its ends. The connector G provided at one end of the collection tube F is fitted into the collection inlet H3 of the cap H and the connector G provided at the other end of the collection tube F is fitted into the fitting portion 9a of the mantle 2 of the bone marrow harvesting needle 1.

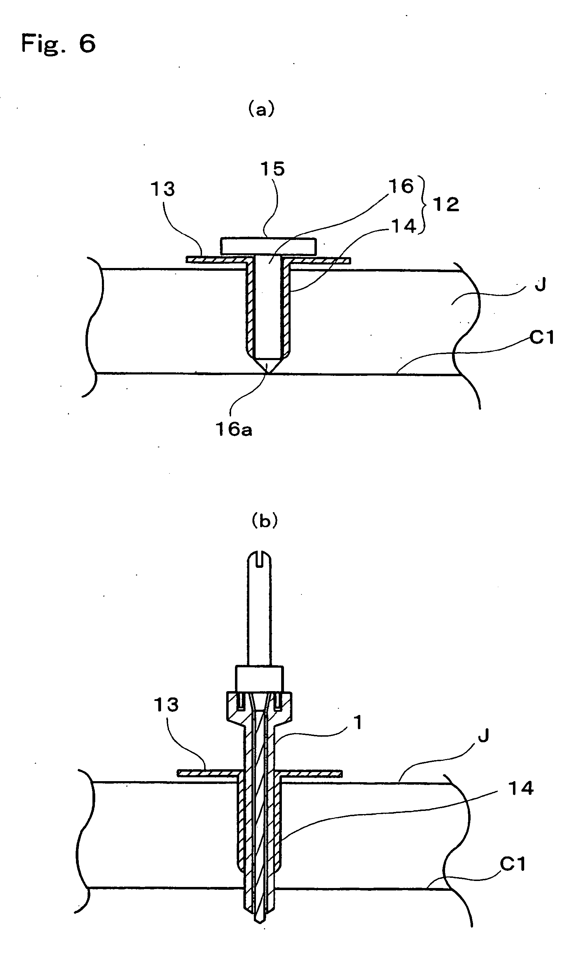

For the harvest of bone marrow, a guide needle 12 shown in FIG. 6(a) is used. The guide needle 12 includes: a guide cylinder 14 with an outside flange 13 and a shaft needle 16 with a head portion 15. Into the guide cylinder 14, the bone marrow harvesting needle 1 can be fitted as shown in FIG.6(b).

In the following, a method will be described for harvesting bone marrow from a long bone C, such as humerus, using the above described needle and set.

First, as shown in FIG. 6(a), skin J is incised with a scalpel, and the guide needle 12 is inserted into the skin from the incised site to allow the tip 16a of the shaft needle 16 thereof to reach a periosteum C1. If the tip of the bone marrow harvesting needle 1 is inserted to reach the periosteum C1 without the guide needle 12, cell tissues are greatly damaged by the inner needle 3 and the cutting edge 21 of the mantle 2. However, the use of the guide needle 12 can prevent such damage.

Further, if the positional relation between the guide needle 12 and the long bone C is checked by CT scan etc. in advance, decision of the site where the bone marrow harvesting needle 1 indwells becomes easy, and moreover, the record can be reserved.

Then, the shaft needle 16 of the guide needle 12 is drawn out from the guide cylinder 14 while leaving the guide cylinder 14 indwelling. Next, the bone marrow harvesting needle 1 having been fitted to the electric drill A is inserted into the guide cylinder 14.

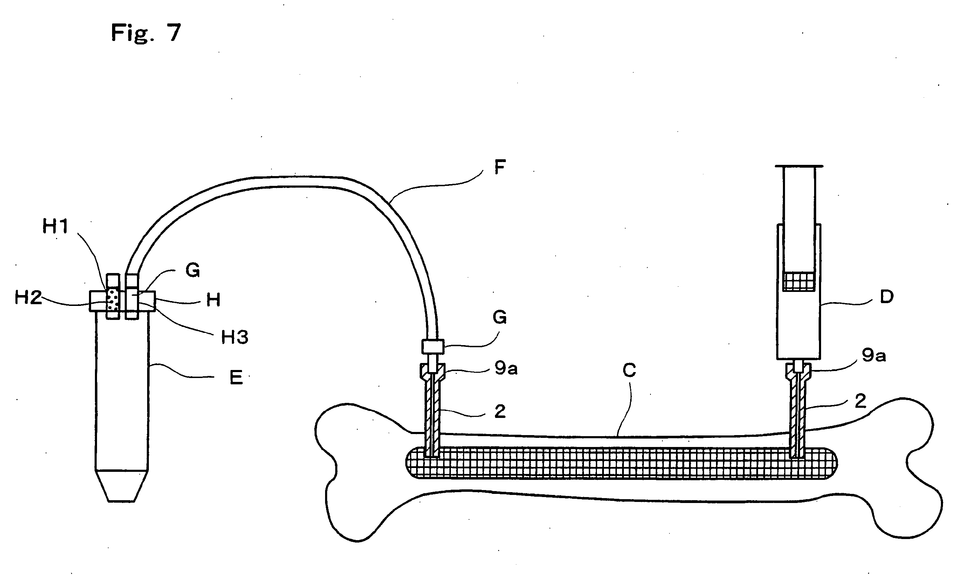

The electric drill A is then rotated, while pressing the bone marrow harvesting needle 1, so as to drill a hole in the long bone C by the use of the inner needle 3 and the cutting edges 21 of the mantle 2. After the tip of the bone marrow harvesting needle 1 reaches bone marrow, the electric drill A is pulled toward a surgeon to draw the inner needle 3 out from the mantle 2 while leaving the mantle 2 indwelling at one end of the long bone C, as shown in FIG. 7. In the other end of the long bone C, another mantle 2 is also allowed to indwell in the same manner as above.

Then, as shown in FIG. 7, an injection orifice of an injection syringe D filled with heparinized saline solution or a tube extending from a parenteral fluid pump or a parenteral fluid pack hung from a parenteral fluid stand to be positoned at an appropriate hight is connected to the fitting portion 9a of one of the mantles 2. And into the fitting portion 9a of the other mantle 2, the connector G at the other end of the collecting tube F is pressed.

Bone marrow is collected into the centrifuge container E through the collection tube F by injecting heparinized saline solution slowly into the long bone C from the injection syringe D, or the parenteral fluid pump or the parenteral fluid pack positioned at a regulated hight. After completing the collection of bone marrow, the connectors G of the collection tube F are detached. The centrifuge container E is capped with a sterilized cap (not shown in the figure). Immediately after this, the collected bone marrow is centrifuged to recover bone marrow cell fractions. Such a through process using the bone marrow harvesting set can minimize the exposure of harvested bone marrow to a non-aseptic environment.

### [Embodiment 2]

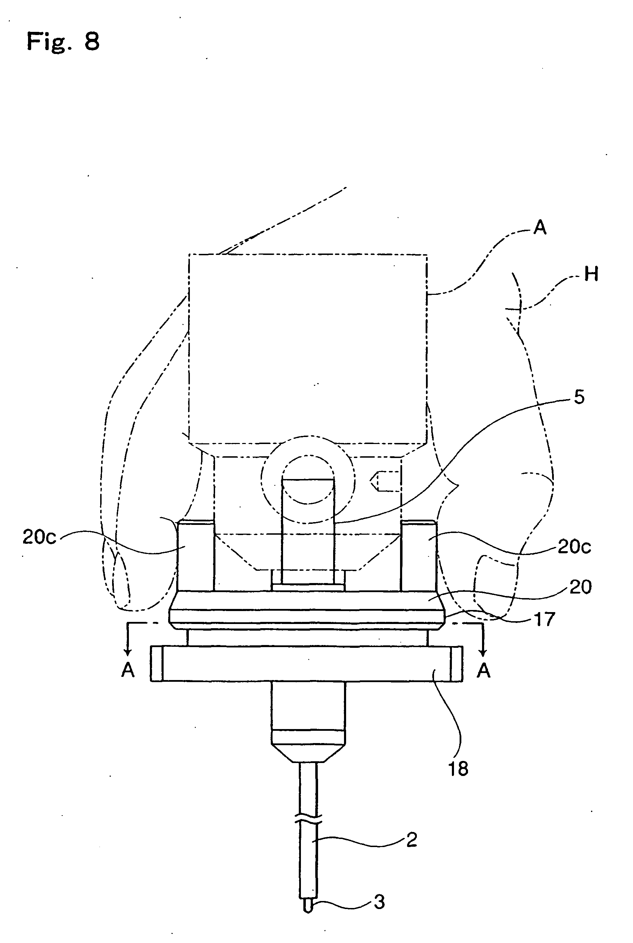

FIGS. 8 to 13 illustrate a bone marrow harvesting set and a bone marrow harvesting needle of embodiment 2. The bone marrow harvesting needle 1 includes: a mantle 2, an inner needle 3 and co-rotation preventive mechanism 17.

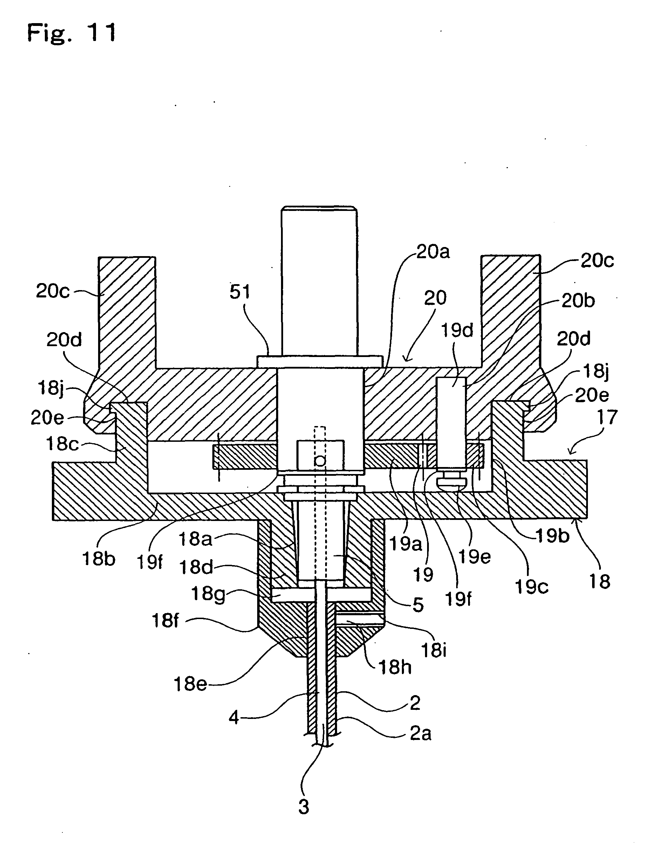

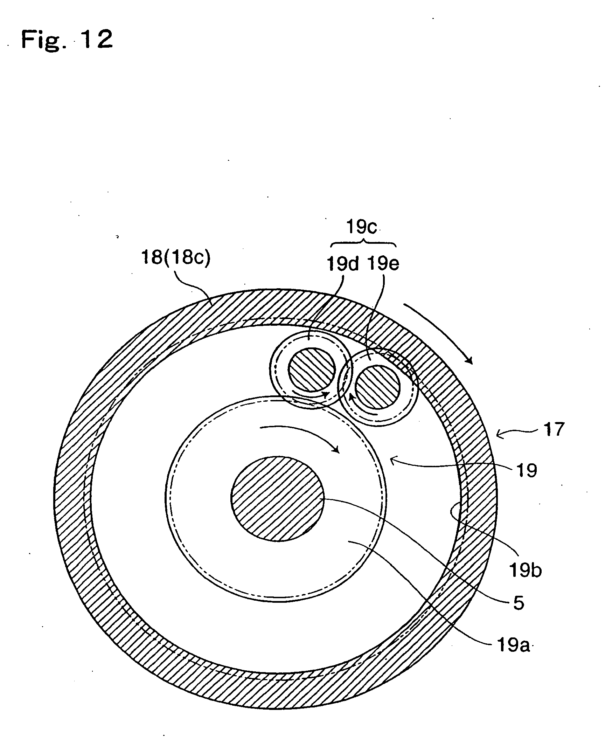

As shown in FIGS. 11 and 12, the co-rotation preventive mechanism 17 includes: a speed reduction mechanism 19; an upper housing 20; and a lower housing 18.

The inner needle body 4 of the inner needle 3 is constructed as described above. As shown in FIG. 11, on the upper end side of the inner needle body 4, a holding shaft 5 is provided.

The mantle body 2a of the mantle 2 is constructed as described above. As shown in FIG. 11, on the upper end side of the mantle body 2a, the lower housing 18 is provided.

The lower housing 18 includes a base portion 18b. The base portion 18b is provided with a central hole. From the peripheral portion of the central hole a cylinder portion 18d is extends downward. On the internal circumference side of the cylinder portion 18d, a tapered bearing bore 18a is formed. On the upper surface of the base portion 18b a circular circumference wall 18c is raised. Outside the circumference wall 18c, four hooked portions 18 j are provided. These hooked portions 18 j are arranged in the circumferential direction of the circumference wall 18c at intervals of 90 degrees.

As shown in FIG. 11, a holding head 18f is fitted on and fixed to the cylinder portion 18d of the lower housing 18. The holding head 18f is provided with a fitting hole 18e and a threaded hole 18i. A threaded screw 18h is screwed into the threaded hole 18i. Into the fitting hole 18e, the upper end portion of the mantle body 2a is inserted, and the mantle body 2a is fixed to the holding head 18f by pressing the tip of the threaded screw 18h against the periphery of the mantle body 2a. Between the tip of the cylinder portion 18d and the holding head 18f, a containing portion 18g for containing cut pieces of a bone C2 is formed.

The speed reduction mechanism 19 includes: a driving gear 19a fixed to the holding shaft 5 of the inner needle 3; an inner teeth portion 19b formed throughout the full circumference of the circumference wall 18c of the lower housing 18 provided on the upper end side of the mantle body 2a; and two intermediate gears 19c engaging with each other. One of the two intermediate gears 19c engages the driving gear 19a and the other engages the inner teeth portion 19b. The driving rotation of the inner needle 3 is transmitted to the mantle 2 via the speed reduction mechanism 19, causing the rotational speed of the mantle 2 to be reduced. The gears 19a and 19c are molded products of resin, such as polyacetal. And the lower housing 18 and the upper housing 20 are formed of, for example, ABS resin or polyacetal resin.

The upper housing 20 is in the form of disc, in its center is formed a bearing bore 20a, on its top surface side is formed a pair of mantle holders 20c, and in its bottom surface side are formed two bearing bores 20b.

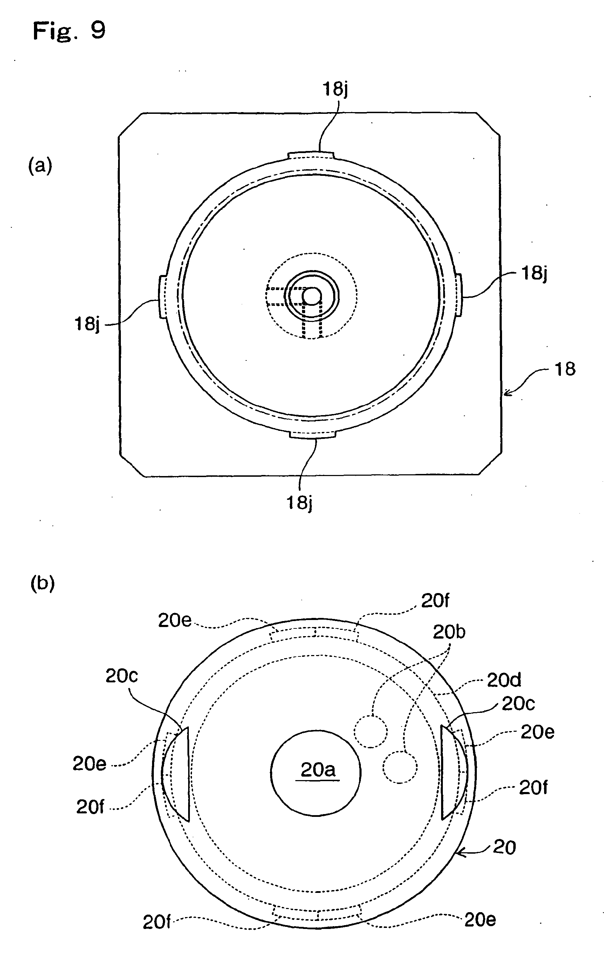

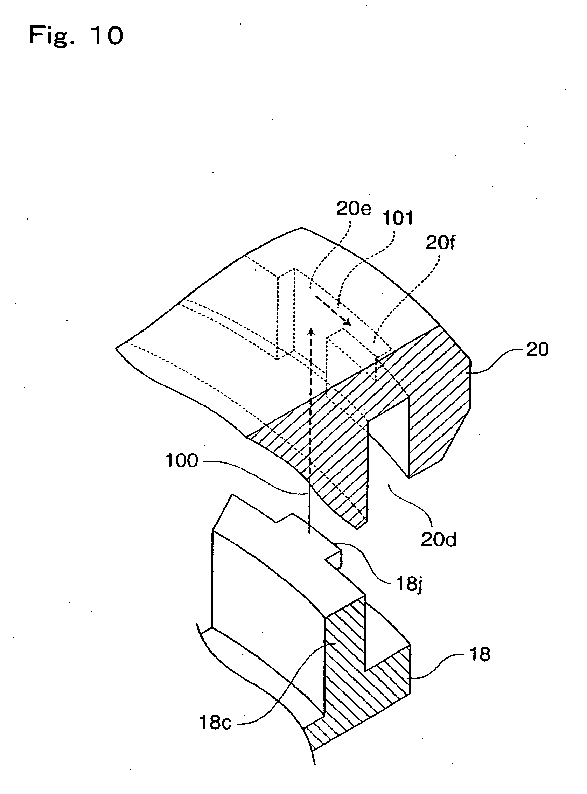

As shown in FIG. 9(b), in the bottom surface side of the peripheral portion of the upper housing 20 a ring groove 20d is formed around the bearing bore 20a. On the inside wall portion of the ring groove 20d, are provided four entrance grooves 20e. These entrance grooves are formed in the circumferential direction thereof at intervals of 90 degrees. As shown in FIG. 10, on the inside wall portion of the ring groove 20d are formed hook grooves 20f in communication with the entrance grooves 20e.

When joining the upper housing 20 and the lower housing 18 together, the inner needle body 3 is inserted into the mantle body 2a, the lower end of the holding shaft 5 of the inner needle 3 is inserted into the bearing bore 18a of the lower housing 18, and the circumference wall 18c of the lower housing 18 is inserted into the ring groove 20d of the upper housing 20. And, after the hook portions 18j of the lower housing 18 is inserted into the entrance grooves 20e of the upper housing 20 as shown by an arrow 100 of FIG. 10, the hook portions 18j are moved to the hook grooves 20f as shown by an arrow 101. When releasing the join of the upper housing 20 and the lower housing 18, the operation is reversed.

The upper end of the holding shaft 5 of the inner needle 3 is inserted into the bearing bore 20a of the upper housing 20 rotatably. The shaft bodies 19d of the intermediate gears 19c are pressed into the bearing bores 20b of the upper housing 20, and the falling of the intermediate gears 19c from the shaft bodies 19d is prevented by an E ring 19f. To the holding shaft 5 is fitted a slip-off preventive clamp 51. On each shaft body 19d is formed a hemispherical head portion 19e, and the head portions 19e are in contact with the lower housing 18. The gear 19a is also prevented from falling from the holding shaft 5 by the E ring 19f.

When drilling a hole in a long bone C using the bone marrow harvesting needle 1 constructed as above, the holding shaft 5 of the inner needle 3 is attached to an electric drill A as shown in FIG. 8, and the inner needle 3 is rotated while fixing the mantle holder 20c with the fingers of a hand H as shown by two-dots-dash line. This allows the gears 19a, 19b and 19c of the speed reduction mechanism 19 to rotate in the direction shown by the arrows of FIG. 12, causing the rotational speed of the mantle 2 to be reduced, whereby the inner needle body 4 and the mantle body 2a are prevented from co-rotating in the same direction at the same speed.

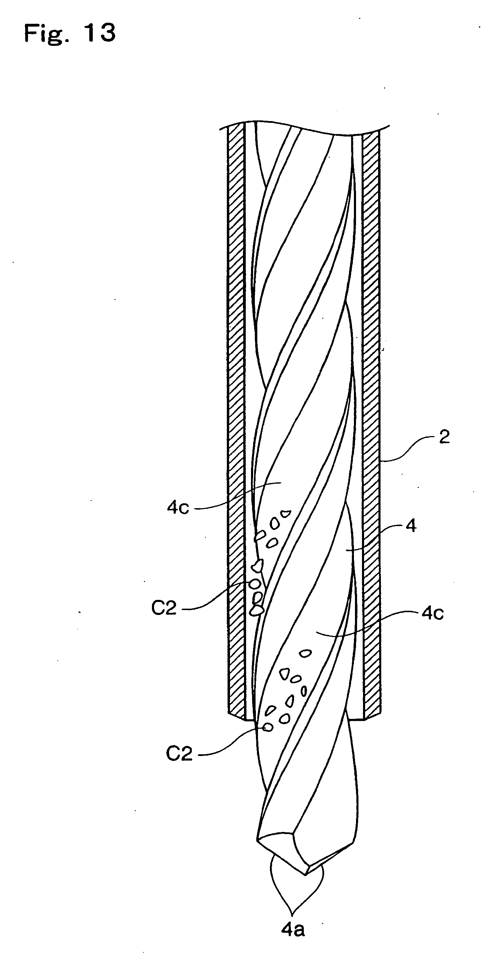

Accordingly, the inner needle body 4 and the mantle body 2a are allowed to function as a screw and as a trough, respectively. Thus, fine cut pieces of a bone C2 having been cut by the inner needle body 4 and the mantle body 2a, as shown in FIG. 13, are conveyed upward along the helical grooves 4c of the inner needle body 4 on the conveyance principle of a screw conveyer and contained in the containing portion 18g of the mantle body 2a.

The cutting edges 4a and 21 of the inner needle body 4 and the mantle body 2a shown in FIGS. 1 and 2, respectively, bite into the long bone C and cut the same. The cut pieces of the bone C2 cut by the inner needle body 4 are discharged along the helical grooves 4c of the inner needle body 4. The cut pieces of the bone C2 cut by the cutting edge 21 of the mantle body 2a are pushed out radial inside of the mantle body 2a by the rake face 21b of the cutting edge 21 and thereby discharged along the helical groove 4c of the inner needle body 4. The inner needle 3 and the mantle 2 are rotated at a low speed so as not to damage the fatty tissues.

After completing the operation, the join of the upper housing 20 and the lower housing 18 is released by holding the lower housing 18 in one hand and the upper housing 20 in the other hand, then the inner needle body 4 is drawn from the mantle 2 by pulling the upper housing 20 up to leave the mantle body 2a indwelling in the bone.

In order to avoid the chances of infections as much as possible, the bone marrow harvesting needle 1 is used only once and thrown away.

### [Variation]

In a alternative embodiment, a bone marrow harvesting needle may have the angles 62 and θ3 of the cutting edge of the mantle body of 0 degree, and the inner needle body and the mantle body may be co-rotated in the same direction at the same speed.

### [Example]

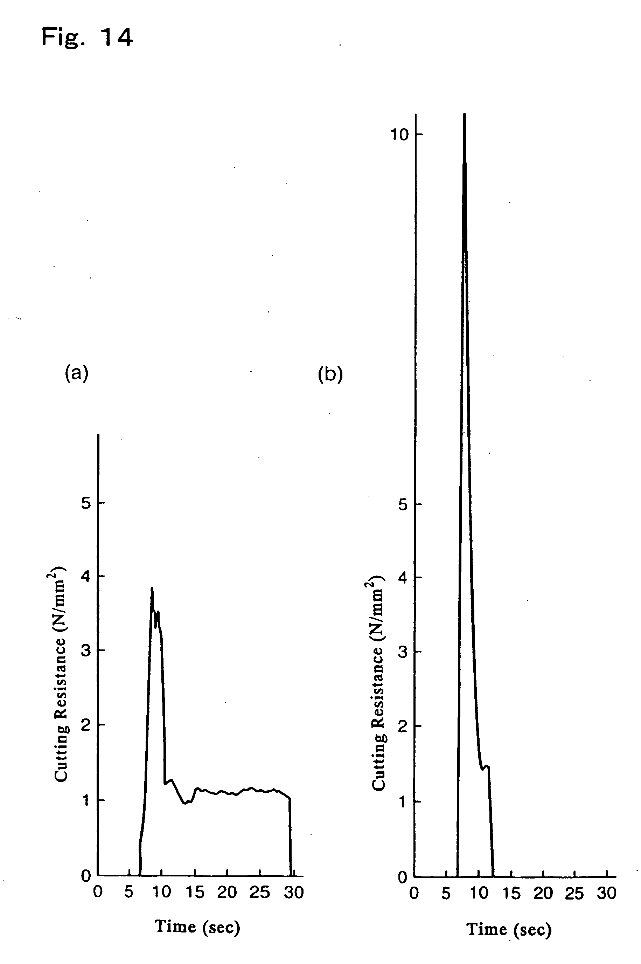

FIGS. 14(a) and 14(b) show the measured results of cutting force when making boring in a material to be subjected to cutting. As a material to be subjected to cutting was used a polyacetal (Juracon) rod, and the rotation ratio of the inner needle 3 of the bone marrow harvesting needle 1 to the mantle body 2 of the same was set to 1:0.5. In another example, the bone marrow harvesting needle of the above-mentioned alternative embodiment was used (the diameter of the needles were both 2 mm). Boring was made while setting the rotational speed of the electric drill to 570 rpm.

As a result, the cutting force of the latter example was 30 N (10 N/mm²) or more at peak, as shown in FIG. 14(b), and that of the former example was 12 N (about 3.75N/mm²) at peak and got in equilibrium at about 3 N (about 1 N/mm²) after peak. These results revealed that in both of the bone marrow harvesting needles of the examples, cutting force was held down and drilling operation was stabilized, however, the needle of the former example was improved compared with that of the latter example.

These embodiments are intended to illustrate the invention and the invention is not intended to be limited to the specific embodiments.

## Claims

1. A bone marrow harvesting set comprising: a centrifuge container (E) for harvesting bone marrow; a cap (H) with a collection inlet through which bone marrow is collected; and a collection tube (F) with connectors (G) attached to its respective ends; wherein the bone marrow harvesting set is constructed in such a manner that the centrifuge container (E) is capped with the cap (H); one of the connectors (G), attached to one end of the collecting tube (F), is adapted to be fitted snugly into the collection inlet (H3) of the cap (H); and the other connector (G), attached to the other end of the collecting tube (F), is adapted to be fitted snugly into a mantle (2) of a bone marrow harvesting needle (1) to be kept indwelling in bone marrow.

2. The bone marrow harvesting set according to claim 1, wherein the cap (H) is provided with an air vent (H2) to equalize the internal and external pressures of the centrifuge container (E) and the air vent (H2) is mounted with a filter (H1).

3. The bone marrow harvesting set according to claim 1 or 2, wherein the mantle of the bone marrow harvesting needle is tubular and the bone marrow harvesting needle comprises: an inner needle (3) which is inserted into and detachably attached to the mantle (2) with its tip adapted to project from the mantle, wherein the inner needle (3) has a drilling edge (4a) formed at its tip and a helical groove (4c) formed on its periphery, and the mantle (2) has a cutting edge (21) formed at its tip.

4. The bone marrow harvesting set according to claim 3, wherein a co-rotation prevention mechanism is provided for preventing the mantle (2) from co-rotating with the inner needle (3).

5. The bone marrow harvesting set according to claim 4, wherein the co-rotation prevention mechanism (17) is constructed in such a manner that rotational power produced by a rotation driving source (A) is transmitted to the concentrically positioned inner needle (3) and mantle (2) and the inner needle and the mantle are rotated at different angular velocities.

6. The bone marrow harvesting set according to claim 4 or 5, wherein the co-rotation prevention mechanism (17) comprises a speed reduction mechanism (19) which allows the mantle (2) to rotate at a reduced speed by transmitting the rotation of the inner needle (3) to the mantle (2).

7. The bone marrow harvesting set according to claim 3, wherein the inner needle (3) is provided with a holder (5), which is adapted to be gripped by chuck claws (A1) of a drill (A), and at the rear end of the holder a fitting portion (10) is formed for fitting a handle (B).

## Patentansprüche

1. Knochenmarkentnahmesatz, umfassend: einen Zentrifugenbehälter (E), um Knochenmark zu entnehmen; einen Deckel (H) mit einem Sammeleinlass, durch den Knochenmark eingesammelt wird; und eine Sammelröhre (F) mit Verbindungen (G), die jeweils an ihren Enden angebracht sind; wobei der Knochenmarkentnahmesatz auf eine solche Weise konstruiert ist, dass der Zentrifugenbehälter (E) mit dem Deckel (H) bedeckt ist; eine der Verbindungen (G), die an einem Ende der Sammelröhre (F) angebracht ist, angepasst ist, um gut in den Sammeleinlass (H3) des Deckels (H) zu passen; und die andere Verbindung (G), die an dem anderen Ende der Sammelröhre (F) angebracht ist, angepasst ist, um gut in eine Hülle (2) einer Knochenmarkentnahmenadel (1) zu passen, die in Knochenmark verbleibt.

2. Knochenmarkentnahmesatz nach Anspruch 1, wobei der Deckel (H) mit einer Luftöffnung (H2) versehen ist, um den inneren und äußeren Druck des Zentrifugenbehälters (E) auszugleichen, und die Luftöffnung (H2) mit einem Filter (H1) montiert ist.

3. Knochenmarkentnahmesatz nach Anspruch 1 oder 2, wobei die Hülle der Knochenmarkentnahmenadel röhrenförmig ist und die Knochenmarkentnahmenadel umfasst: eine innere Nadel (3), die in die Hülle (2) eingesetzt und abnehmbar daran angebracht ist, wobei ihre Spitze angepasst ist, um von der Hülle vorzustehen, wobei die innere Nadel (3) eine Bohrkante (4a), die an ihrer Spitze ausgebildet ist, und eine Schraubennut (4c), die an ihrem Umfang ausgebildet ist, umfasst und die Hülle (2) eine Schneidkante (21) umfasst, die an ihrer Spitze ausgebildet ist.

4. Knochenmarkentnahmesatz nach Anspruch 3, wobei ein Ko-Rotations-Verhinderungsmechanismus vorgesehen ist, um die Hülle (2) daran zu hindern, mit der inneren Nadel (3) zusammen zu rotieren.

5. Knochenmarkentnahmesatz nach Anspruch 4, wobei der Ko-Rotations-Verhinderungsmechanismus (17) auf eine solche Weise konstruiert ist, dass eine Rotationsleistung, die durch eine Rotationsantriebsquelle (A) erzeugt wird, auf die konzentrisch positionierte innere Nadel (3) und die Hülle (2) übertragen wird und die innere Nadel und die Hülle mit verschiedenen Winkelgeschwindigkeiten rotiert werden.

6. Knochenmarkentnahmesatz nach Anspruch 4 oder 5, wobei der Ko-Rotations-Verhinderungsmechanismus (17) einen Geschwindigkeitsreduktionsmechanismus (19) umfasst, der es der Hülle (2) durch Übertragen der Rotation der inneren Nadel (3) auf die Hülle (2) erlaubt, sich mit einer reduzierten Geschwindigkeit zu drehen.

7. Knochenmarkentnahmesatz nach Anspruch 3, wobei die innere Nadel (3) mit einem Halter (5) versehen ist, der angepasst ist, um durch Spannbacken (A1) eines Bohrers (A) ergriffen zu werden, und an dem rückseitigen Ende des Halters ein Befestigungsabschnitt (10) zum Befestigen eines Griffs (B) ausgebildet ist.

## Revendications

1. Ensemble d'échantillonnage de moelle osseuse comprenant : un récipient centrifuge (E) pour échantillonner la moelle osseuse ; un bouchon (H) doté d'une entrée de collecte à travers laquelle la moelle osseuse est collectée ; et un tube de collecte (F) doté de raccords (G) fixés à ses extrémités respectives ; dans lequel l'ensemble d'échantillonnage de moelle osseuse est construit de telle manière que le récipient centrifuge (E) est coiffé avec le bouchon (H) ; un des raccords (G), fixé à une extrémité du tube de collecte (F), est adapté de sorte à être ajusté étroitement dans l'entrée de collecte (H3) du bouchon (H) ; et l'autre raccord (G), fixé à l'autre extrémité du tube de collecte (F), est adapté de sorte à être ajusté étroitement dans un manchon (2) d'une aiguille d'échantillonnage de moelle osseuse (1) pour être maintenu à demeure dans la moelle osseuse.

2. Ensemble d'échantillonnage de moelle osseuse selon la revendication 1, dans lequel le bouchon (H) est doté d'un évent (H2) pour égaliser les pressions interne et externe du récipient centrifuge (E) et l'évent (H2) est équipé d'un filtre (H1).

3. Ensemble d'échantillonnage de moelle osseuse selon la revendication 1 ou 2, dans lequel le manchon de l'aiguille d'échantillonnage de moelle osseuse est tubulaire et l'aiguille d'échantillonnage de moelle osseuse comprend : une aiguille interne (3) qui est insérée dans le manchon (2) et fixée de façon détachable à celui-ci, sa pointe étant adaptée pour faire saillie hors du manchon, dans lequel l'aiguille interne (3) présente un bord de forage (4a) formé au niveau de sa pointe et une rainure hélicoïdale (4c) formée sur sa périphérie, et le manchon (2) présente un bord de découpe (21) formé au niveau de sa pointe.

4. Ensemble d'échantillonnage de moelle osseuse selon la revendication 3, dans lequel un mécanisme de prévention de co-rotation est prévu pour empêcher le manchon (2) de tourner conjointement avec l'aiguille interne (3).

5. Ensemble d'échantillonnage de moelle osseuse selon la revendication 4, dans lequel le mécanisme de prévention de co-rotation (17) est construit de telle manière que la puissance de rotation produite par une source d'entraînement en rotation (A) est transmise à l'aiguille interne positionnée de façon concentrique (3) et au manchon (2) et l'aiguille interne et le manchon tournent à différentes vitesses angulaires.

6. Ensemble d'échantillonnage de moelle osseuse selon la revendication 4 ou 5, dans lequel le mécanisme de prévention de co-rotation (17) comprend un mécanisme de réduction de vitesse (19) qui permet au manchon (2) de tourner à une vitesse réduite en transmettant la rotation de l'aiguille interne (3) au manchon (2).

7. Ensemble d'échantillonnage de moelle osseuse selon la revendication 3, dans lequel l'aiguille interne (3) est dotée d'un support (5), qui est adapté pour être saisi par les griffes de serrage (A1) d'un foret (A), et sur l'extrémité arrière du support, est formée une partie de fixation (10) pour fixer une poignée (B).
